# EUROPÄISCHE PATENTSCHRIFT

(11) **EP 0 586 335 B1**
(45) Veröffentlichungstag und Bekanntmachung des Hinweises auf die Patenterteilung: **03.05.2000**
(21) Anmeldenummer: 93810560.8
(22) Anmeldetag: 09.08.1993
(51) Int. Cl.: A61F 2/34

(54) **Zweiteilige Hüftgelenkpfanne**
Two-piece acetabular cup
Coque acétubulaire en deux parties

(30) Priorität: 02.09.1992 EP 92810669
(43) Veröffentlichungstag der Anmeldung: 09.03.1994
(73) Patentinhaber: Sulzer Orthopädie AG, 6340 Baar (CH)
(72) Erfinder: Spotorno, Lorenzo, Prof. Dr. med., I-17024 Finale Ligure (IT); Koch, Rudolf, CH-8500 Frauenfeld (CH); Willi, Rolang, CH-8413 Neftenbach (CH)
(74) Vertreter: Trieblnig, Adolf

(56) Entgegenhaltungen:
- EP-A- 0 270 744
- EP-A- 0 315 795
- EP-A- 0 351 545
- EP-A- 0 412 438
- EP-A- 0 486 403
- WO-A-85/00284
- WO-A-88/07845
- FR-A- 2 638 963
- FR-A- 2 684 544
- US-A- 5 092 897

## Beschreibung

Die Erfindung betrifft eine Spreizpfanne gemäss dem Oberbegriff des unabhängigen Anspruchs 1.

Die Patentanmeldung EP-A-0 486 403 zeigt eine Spreizschale mit einem konischen Ring, der zum Spreizen der Aussenschale eingeschraubt wird. Eine Innenschale, deren innere Lagerfläche in einem Winkel zur Polachse der Aussenschale geschwenkt ist und durch eine an die Lagerfläche anschliessende, asymmetrische Überhöhung fortgesetzt ist, kann an dem konischen Ring in verschiedenen Drehwinkelstellungen mit einer Schnappverbindung gehalten werden. Ein Nachteil dieser Anordnung besteht darin, dass keine kraftschlüssige Verbindung zwischen Innenschale und Aussenschale besteht. Ein weiterer Nachteil besteht darin, dass wegen der vorstehenden Lagerfläche, welche die Positionierfehler der Aussenschale korrigiert, eine Mindestüberhöhung auf der Gegenseite angebracht sein muss, die keine Relation zu der Richtung hat, in der eine eigentliche Luxation verhindert werden soll.

Hüftgelenkpfannen der vorstehend genannten Art sind beispielsweise aus der EP-B-0 242 633 bekannt. Die Pfanne nach dem genannten Patent ist eine sogenannte Spreizpfanne, deren Aussenschale auf ihrem Umfang von der Basis her geschlitzt ist, so dass einzelne Lappen entstehen. Durch Einschrauben einer Innenschale werden die Lappen, die aussen mit Dornen versehen sind, zur Verankerung der Pfanne aufgeweitet, wobei die Dorne in den Knochen eindringen.

Weiterhin ist es bekannt, eine Hüftgelenkpfanne bzw. einer ihrer Schalen auf einem Teil des Umfangs der äquatorialen Basis mit einer Überhöhung zu versehen, um die Gefahr von Luxationen des Gelenkkopfes bei extremen Auslenkungen des Gelenks zu verringern oder zu vermeiden.

Um eine "korrekte" Einstellung einer einseitig vergrösserten Artikulationsfläche zu ermöglichen, ist in dem EP-Patent 0 150 198 eine zweischalige Pfanne gezeigt, bei der die Innenschale, die die einseitig vergrösserte Artikulationsfläche aufweist, in unterschiedlichen Winkelstellungen in die starre Aussenschale eingesetzt werden kann. Ähnlich kann bei der Pfanne nach dem EP-Patent 0 270 744 eine Innenschale mit einseitig angesetzter Luxationshilfe in verschiedenen Winkelstellungen in eine einschraubbare Aussenschale eingesetzt werden.

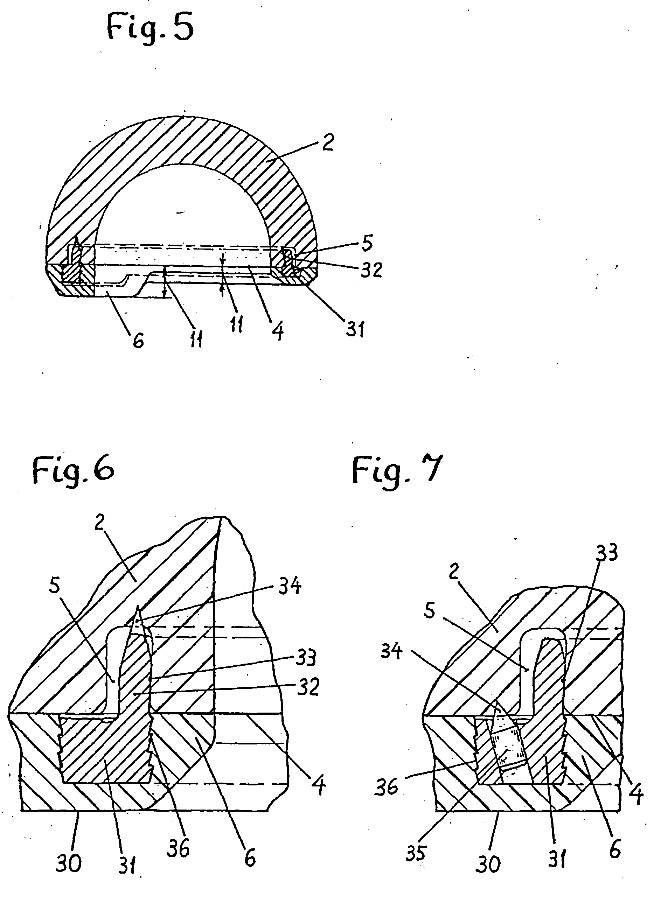

Die US-A-5,092,897 zeigt Innenschalen, deren halbkugelförmige Artikulationsflächen mit ihrer Polachse zur Polachse einer zugehörigen Aussenschale um einen Winkel geneigt ist. Gezeigt werden verschiedene Befestigungsmittel zwischen Innen- und Aussenschale, die eine Drehung der Innenschale mit ihrer schräg stehenden Halbkugelfläche um die Polachse der Aussenschale und anschliessend eine Verankerung der Innenschale mit der Aussenschale ermöglichen. Am Beispiel der Figuren 6 bis 12 wird eine Innenschale gezeigt, die quer zur Polachse durch die halbkugelförmige Artikulationsfläche hindurch zweigeteilt ist, um den äusseren Teil der Artikulationsfläche als geschlitzten Ring auszuführen und um diesen als Sicherungselement für den inneren Teil an der Aussenschale zu verankern.

Da bei einer durch Drehung der Innenschale fixierbaren Aussenschale eine definierte Endstellung der Innenschale nicht gegeben ist, ist es nicht möglich, an der Innenschale angeordnete Luxationshilfen in einer optimalen Winkelstellung im Knochen zu fixieren.

Aufgabe der Erfindung ist es daher, bei Pfannen der eingangs genannten Art eine optimale Winkellage einer Luxationshilfe zu ermöglichen.

Gemäss der vorliegenden Erfindung wird diese Aufgabe durch die Kennzeichen vom unabhängigen Anspruch 1 gelöst.

Bei der neuen Konstruktion ist die Überhöhung ein von der - im allgemeinen rotationssymmetrisch zur Polachse der Pfanne ausgebildeten - Innenschale getrennter Teil. Sie kann intraoperativ mit einer optimalen Winkelstellung in die Innenschale eingesetzt werden, nachdem diese in ihrer Endstellung fixiert worden ist.

Ein zusätzlicher Vorteil der neuen Konstruktion besteht darin, dass Innenschale und Überhöhung aus unterschiedlichen Materialien hergestellt werden können.

Eine kontinuierliche Einstellung der optimalen Winkelstellung wird ermöglicht, wenn die Befestigungselemente in beliebiger Drehwinkelstellung kraftschlüssig verankerbar, zum Beispiel durch Reibung in der Ringnut gehalten, sind.

Unter Verzicht auf eine kontinuierliche Einstellung kann zusätzlich zur oder statt der kraftschlüssigen Verankerung die Überhöhung in Winkelbereichen grösserer Dicke Zapfen aufweisen, die sie formschlüssig in entsprechenden Löchern der Stirnseite der Basisfläche gegen Verdrehung sichern.

Dabei kann eine stufenweise Einstellung der Winkelstellung beispielsweise verwirklicht werden, indem die Löcher mit einer konstanten Winkelteilung über den ganzen Umfang der ringförmigen Basisfläche verteilt sind, wobei ihre Winkelteilung einem ganzzahligen Bruchteil des Winkelabstandes der Zapfen entspricht.

Im folgenden wird die Erfindung anhand von Ausführungsbeispielen im Zusammenhang mit der Zeichnung näher erläutert.
- Fig. 1: zeigt teilweise im Schnitt eine erste Ausführungsform der neuen Hüftgelenkpfanne;
- Fig. 2: gibt in einem Meridianschnitt ein zweites Beispiel wieder;
- Fig. 3: ist eine Aufsicht auf die der Innenschale zugewandte Seite der ringförmigen Überhöhung, gesehen in Richtung der Rotationsachse des Ringes;
- Fig. 4: schliesslich stellt eine Aufsicht in Richtung zum Pol einer Innenschale mit eingesetzter Überhöhung dar, deren Darstellung auf einem Teil des Umfanges unterbrochen ist.

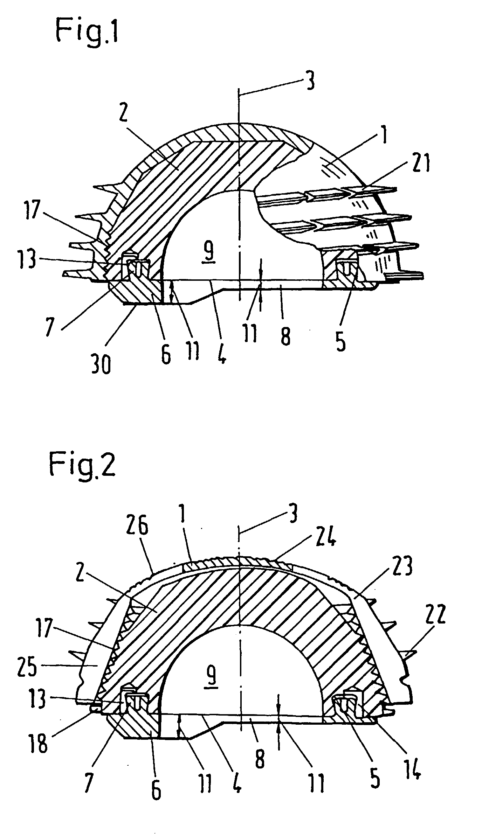

Die vorzugsweise aus Metall gefertigte und bezüglich der Polachse 3 rotationssymmetrische Aussenschale 1 der in Fig. 1 gezeigten Schraubpfanne trägt auf ihrer äusseren Oberfläche ein selbstschneidendes Gewinde 21, mit dem sie sich bei der Implantation in den nicht gezeigten Beckenknochen einschraubt. In ihren polygonförmig begrenzten Hohlraum ist eine Innenschale 2 aus Kunststoff eingesetzt und mit Hilfe eines Gewindes 17 fixiert.

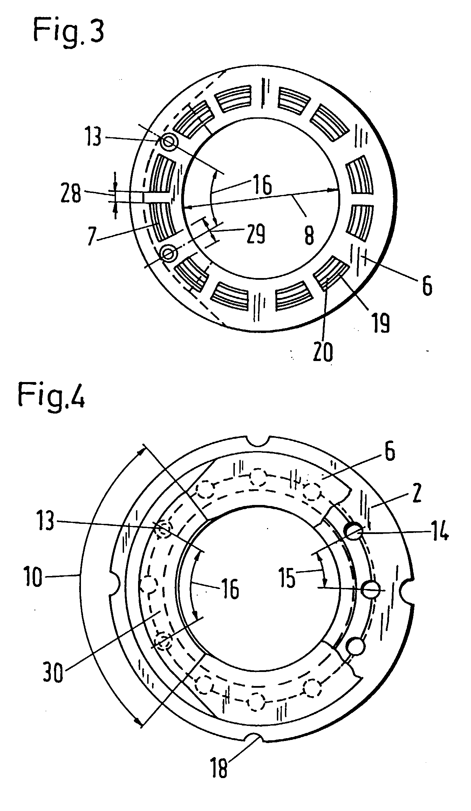

Die Innenschale 2 hat in ihrer stirnseitigen Basisfläche 4, die eine zur Aufnahme des Gelenkkopfes dienende Pfannenschale 9 ringförmig umschliesst, eine hinterschnittene Ringnut 5. In diese ist eine Überhöhung 6 eingesetzt und durch, ebenfalls mit Hinterschneidungen versehene Befestigungselemente 7 gehalten; die Befestigungselemente 7 sind im vorliegenden Beispiel als ringsektorfömige Abschnitte ausgebildet (Fig. 3), die unterschiedliche Abstände 28 und 29 voneinander haben. Ein einzelner Abschnitt der Befestigungselemente 7 besteht aus zwei durch einen Schlitz 20 voneinander getrennten, parallel zueinander verlaufenden Zungen 19 (Fig. 3). Aussen an ihrer Basis hat die Innenschale 2 vier - um einen rechten Winkel gegeneinander versetzte Aussparungen 18, in die für das Einschrauben der Innenschale 2 in die Aussenschale 1 ein nicht gezeigtes Instrument eingesetzt wird.

Bei der Konstruktion nach Fig. 2 handelt es sich um eine sogenannte Spreizpfanne; die Aussenschale 1 dieser Pfanne weist um die Polachse 3 herum einen kompakten Mittelteil 24 auf, von dem aus sich - durch in Meridianlinien verlaufende Einschnitte getrennt - einzelne lappenartige Schalensegmente 23 zur Basisfläche 4 erstrecken. Die Segmente 23 bestehen aus einer relativ dünnwandigen, an den Mittelteil 24 anschliessenden, elastischen Zone 26 und einem dickwandigen Gewindeteil 25, der auf seiner äusseren Oberfläche Dorne 22 trägt. Auf der Innenseite des Gewindeteiles 25 ist, ähnlich wie bei der Pfanne nach Fig. 1, das Gewinde 17 eingeschnitten, in das bei der Implantation sich mit ihrem gleichartigen Gewinde wiederum die Innenschale 2 einschraubt. Bei diesem Einschrauben der Innenschale 2 werden die Schalensegmente 23 aufgeweitet, an den Beckenknochen angepresst und so fixiert, wobei die Dorne 22 in den Knochen eindringen.

Im Basisbereich ist die Innenschale 2 nach Fig. 2 gleich aufgebaut wie die vorstehend beschriebene nach Fig. 1.

Bei dieser Konstruktion besitzt die Überhöhung 6, deren Ringöffnung 8 an die Basisöffnung der eigentlichen Pfannenschale 9 angepasst ist, entlang ihres Umfangs Winkelbereiche mit unterschiedlichen Dicken. Während der sich über den grösseren Teil des Umfanges erstreckende Winkelbereich mit geringer Dicke lediglich als Abdeckung für die Ringnut 5 dient, hat der Winkelbereich 10 eine demgegenüber grössere Dicke 11 mit einer erhöhten Stirnfläche 30. Wie eingangs geschildert, hat diese erhöhte Stirnfläche 30 die Aufgabe, die Gefahr von Luxationen des Gelenkkopfes bei extremen Auslenkungen zu verringern oder zu vermeiden.

Für eine stufenlose Einstellung einer optimalen Winkelstellung des Winkelbereiches 10 werden die bereits beschriebenen Zungen 19 kraftschlüssig, also lediglich durch Reibung in der Ringnut 5 gehalten, wobei zum Zwecke einer verbesserten Haftung die Flanken ihrer Hinterschneidung - und/oder diejenigen der Ringnut 5 - aufgerauht sein können.

Wird auf eine stufenlose Einstellbarkeit verzichtet, so kann zusätzlich zur oder statt der kraftschlüssigen Verbindung eine formschlüssige Sicherung gegen Verdrehen der Überhöhung 6 vorgesehen sein. Bei der gezeigten Konstruktionen besteht die formschlüssige Verbindung aus zwei, im Winkelbereich 10 im Winkelabstand 16 (Fig. 4) voneinander angeordneten Zapfen 13.

Die Zapfen 13 greifen in Löcher 14 ein, die in die Basisfläche 4 der Innenschale 2 eingearbeitet sind. Eine Einstellbarkeit in Stufen wird dabei dadurch erreicht, dass die über den ganzen Umfang der Basisfläche 4 gleichmässig verteilten Löcher 14 eine Winkelteilung 15 haben, die einem ganzzahligen Bruchteil des Winkelabstandes 16 der Zapfen 13 entspricht. Bei dem gezeigten Beispiel ist diese Winkelteilung 15 gleich dem halben Winkel des Winkelabstandes 16.

Wie aus der Schraffur der Fig. 1 und 2 ersichtlich bestehen die Innenschale 2, die im allgemeinen aus Kunststoff gefertigt ist, und die - hier metallene - Überhöhung 6 aus verschiedenen Werkstoffen; durch die Auswahl unterschiedlicher Werkstoffe werden, falls erwünscht oder notwendig, verbesserte Eigenschaften, beispielsweise bezüglich Festigkeit, Reibungsverhalten und/oder Verschleiss, erzielt.

Kurz zusammengefasst stellt sich die Erfindung also folgendermassen dar:

Die in eine Aussenschale 1 einschraubbare Innenschale 2 einer zweiteiligen Spreizpfanne ist auf ihrer Basisfläche 4 mit einer hinterschnittenen Ringnut 5 versehen. In der Ringnut 5 ist eine ringförmige Überhöhung 6 als Luxationshilfe gehalten, die entlang ihres Umfanges mindestens einen Winkelbereich 10 mit einer - relativ zu den restlichen Winkelbereichen - grösseren Dicke 11 aufweist. Die Winkellage des Bereiches 10 grösserer Dicke 11 ist auf dem Umfang, zumindest stufenweise einstellbar.

Die Einstellbarkeit des Winkelbereiches 10 grösserer Dicke 11 in seiner Winkelstellung relativ zur Innenschale 2 ermöglicht eine Optimierung der Stellung dieses Bereiches 10 auf dem Umfang der Innenschale 2, unabhängig von der Endstellung der Innenschale 2, deren Endstellung infolge der Fixierung mit Hilfe des Gewindes 17 nicht exakt definiert ist.

Eine weitere Ausführungsform, bei der eine formschlüssige Verbindung bezüglich Drehung stufenlos zwischen Innenschale 2 und der ringförmigen Ueberhöhung 6 einstellbar ist, wird in den Figuren 5, 6, 7 gezeigt. In der ringförmigen Ueberhöhung 6 ist ein Metallring 31 - vorzugsweise aus Titan - eingepresst und durch Vor- und Rücksprünge 36 gesichert. Er verleiht der ringförmigen Ueberhöhung 6 Stabilität und bildet gleichzeitig mit einem in die Ringnut 5 vorstehenden Kragen 32 eine kraftschlüssige Schnappverbindung 33. Beim Einschnappen dieser Schnappverbindung in der Richtung der Polachse bohrt sich mindestens eine aus dem Metallring 31 vorstehende Spitze 34 in den Rand der Innenschale 2 und stellt so bezüglich Drehung einen Formschluss her. In Figur 6 ist die Spitze 34 als Bestandteil vom Kragen 32 ausgeführt, während in Figur 7 die Spitze 34 mit einem in den Metallring eingepressten Stift erzeugt wird.

## Patentansprüche

1. Spreizpfanne mit einer aufspreizbaren Aussenschale (1), die durch Eindrehen eines ringförmigen Körpers mit konischem Gewinde (17) im Beckenknochen verklemmbar ist, und die eine Innenschale (2) aufweist, welche mit ihrer halbkugelförmigen Lagerfläche und einer daran angeformten, ungleichmässig an deren Umfang vorstehenden ringförmigen Überhöhung (6) in verschiedenen Drehwinkelstellungen relativ zur Aussenschale (1) befestigbar ist, dadurch gekennzeichnet, dass das konische Gewinde (17) direkt an der Innenschale (2) angeformt ist, dass die Innenschale stirnseitig parallel zur Aussenschale (1) ausgerichtet ist und eine ringförmige Basisfläche (4) mit hinterschnittener Ringnut (5) aufweist, in der mit Befestigungselementen (7) eine separate, durchgehend ringförmige Überhöhung (6) mit einem Winkelbereich (10) grösserer Dicke (11) fixiert ist, wobei die Überhöhung (6) zür Verringerung der Luxation eines Gelenkkopfes nach dem Fixieren der Lagerschale in ihrer Endstellung befestigbar ist.

2. Hüftgelenkpfanne nach Anspruch 1, dadurch gekennzeichnet, dass die Befestigungselemente (7) in beliebiger Drehwinkelstellung an der Innenschale (2) gegen Drehung über eine mit Hinterschneidungen versehene Schnappverbindung kraftschlüssig verankerbar sind.

3. Hüftgelenkpfanne nach Anspruch 1 oder 2, dadurch gekennzeichnet, dass die Überhöhung (6) in Winkelbereichen (10) grösserer Dicke (11) Zapfen (13) aufweist, die sie formschlüssig in entsprechenden Löchern (14) der Stirnseite der Basisfläche (4) gegen Verdrehung sichern.

4. Hüftgelenkpfanne nach Anspruch 3, dadurch gekennzeichnet, dass die Löcher (14) mit einer konstanten Winkelteilung (15) über den ganzen Umfang der ringförmigen Basisfläche (4) verteilt sind, wobei
ihre Winkelteilung (15) einem ganzzahligen Bruchteil des Winkelabstandes (16) der Zapfen (13) entspricht.

## Claims

1. An expanding cup having an outer shell (1) capable of expanding, which can be clamped in the pelvis bone by means of screwing-in an annular body having a thread (17), and which has an inner shell (2) which can be secured with its hemi-spherical bearing surface and an annular-shaped superelevation (6) moulded on same, projecting unevenly on the periphery of which, in various angles of rotation positions relative to outer shell (1), characterised in that the conical thread (17) is directly moulded on the inner shell (2), that the inner shell is arranged end-side parallel to outer shell (1) and has an annular-shaped base surface (4) having an undercut annular groove (5), in which a separate, continuous annular-shaped superelevation (6) having securing elements (7) is secured, having an angle area (10) of greater thickness (11), and where the superelevation (6) can be secured for reducing dislocation of a joint head after securing the bearing shell in its end position.

2. An acetabular cup according to claim 1, characterised in that the securing elements (7) can be force-lockingly anchored in any angle of rotation position on inner shell (2) to counter rotation via a snap-fastening provided with undercuts.

3. An acetabular cup according to claims 1 or 2, characterised in that superelevation (6) has pegs (13) in the angle area (10) of greater thickness (11), which protect them form-lockingly from rotation in corresponding holes (14) in the end side of base surface (4).

4. An acetabular cup according to claim 3, characterised in that holes (14) having a constant sub-division of an angle (15), are distributed over the whole periphery of the annular-shaped base surface (4), where its sub-division of an angle (15) corresponds to an integral fraction of the angular distance (16) of the peg (13).

## Revendications

1. Coque d'écartement comportant une coque extérieure (1) pouvant être écartée qui peut être serrée en vissant un corps annulaire à filetage conique (17) dans l'os du bassin, qui présente une coque intérieure (2) qui peut être fixée avec sa face de palier semi-sphérique et une surélévation annulaire (6) rapportée par formage à celle-ci, faisant saillie irrégulièrement à son pourtour, dans différentes positions d'angle de rotation relativement à la coque extérieure (1), caractérisée en ce que le filetage conique (17) est formé directement sur la coque intérieure (2), en ce que la coque intérieure est orientée au côté frontal parallèlement à la coque extérieure (1) et présente une face de base annulaire (4) avec une rainure annulaire (5) à contre-dépouille dans laquelle est fixée avec des éléments de fixation (7) une surélévation annulaire continue séparée (6) avec une zone angulaire (10) de plus grande épaisseur (11), où la surélévation (6), pour réduire la luxation d'une surface articulaire convexe, après la fixation de la coque de palier, peut être fixée dans sa position finale.

2. Coque acétabulaire selon la revendication 1, caractérisée en ce que les éléments de fixation (7) peuvent être ancrés par liaison par force dans une position d'angle de rotation sélective à la coque intérieure (2) à l'encontre d'une rotation par une liaison à enclenchement pourvue de contre-dépouilles.

3. Coque acétabulaire selon la revendication 1 ou 2, caractérisée en ce que la surélévation (6) présente dans des zones angulaires (10) de plus grande épaisseur (11) des tenons (13) qui l'assurent par concordance des formes dans des trous correspondants (14) du côté frontal de la face de base (4) à l'encontre d'une rotation.

4. Coque acétabulaire selon la revendication 3, caractérisée en ce que les trous (14) sont répartis selon une subdivision angulaire constante (15) sur tout le pourtour de la face de base annulaire (4), où leur subdivision angulaire (15) correspond à une fraction entière de l'écart angulaire (16) des tenons (13).
